# EUROPEAN PATENT SPECIFICATION

(11) **EP 4 247 479 B1**
(45) Date of publication and mention of the grant of the patent: **25.09.2024**
(21) Application number: 21819179.9
(22) Date of filing: 19.11.2021
(51) Int. Cl.: A61N 1/05, A61B 1/00

(54) **DEVICES FOR TREATING DISEASE USING ELECTRICAL STIMULATION**
VORRICHTUNGEN ZUR BEHANDLUNG VON KRANKHEITEN MITTELS ELEKTRISCHER STIMULATION
DISPOSITIFS DE TRAITEMENT DE MALADIE À L'AIDE D'UNE STIMULATION ÉLECTRIQUE

(30) Priority: 19.11.2020 US 202063198882 P
(43) Date of publication of application: 27.09.2023
(73) Proprietor: Phagenesis Limited, Manchester, M15 6SE (GB)
(72) Inventor: MULROONEY, Conor, Manchester Science Park Manchester, M15 6SE (GB)
(74) Representative: Stratagem IPM Limited
(86) International application number: PCT/GB2021/053010
(87) International publication number: WO 2022/106843

(56) References cited:
- WO-A1-2009/154718
- US-A- 5 179 952
- US-A1- 2017 224 986
- US-A1- 2019 134 389

## Description

### TECHNICAL FIELD

The invention provides a device according to claim 1. The scope of the invention is defined by the claims. For better understanding of the invention the present disclosure provides further examples of devices, systems, and methods, said methods not forming part of the invention, for treating disease using electrical stimulation. Particular embodiments include treating dysphagia by applying electrical stimulation to a target neural population of the patient.

### BACKGROUND

Dysphagia is the condition whereby a patient has difficulty in swallowing or is unable to swallow safely. Dysphagia may be caused, for example, by stroke, neurodegenerative diseases, brain tumors or in some cases by other co-morbidities, such as respiratory disorders. It has been reported that between 7 and 10% of all adults older than 50 years of age present with clinically significant dysphagia. Of those over the age of 60, this increases to 14%. In total, 10 million Americans are evaluated each year in clinics and hospitals for swallowing difficulties. It has also been reported that over 51% of institutionalized elderly patients present with oropharyngeal dysphagia.

Swallowing is a rigidly ordered sequence of events that results in the propulsion of food from the mouth through the pharynx and esophagus to the stomach. At the same time, respiration is inhibited and food is prevented from entering into the trachea. Swallowing can be initiated voluntarily, but thereafter it is almost entirely under reflex control. The swallow reflex is typically initiated by sensory impulses from tactile receptors (particularly those located near the opening of the pharynx) being transmitted to certain areas in the medulla. The central integrating areas for swallowing lie in the medulla and lower pons; they are collectively called the swallowing center. Motor impulses travel from the swallowing center to the musculature of the pharynx and upper esophagus via various cranial nerves. This lower swallowing center in the brainstem is under regulatory control by higher center in the cerebral cortex. These higher swallowing centers or regions control the voluntary initiation and modulation of the swallow.

Swallowing occurs in three stages. In the oral or voluntary phase, food is moved towards the back of the mouth by the tongue, and forced into the pharynx, where it stimulates the tactile receptors that initiate the swallowing reflex. In the pharyngeal stage of swallowing, food passes through the pharynx by constriction of the walls of the pharynx, backward bending of the epiglottis, and an upward and forward movement of the larynx and trachea. During the pharyngeal stage, respiration is reflexively inhibited. In the esophageal stage of swallowing, food moves down the esophagus and into the stomach, assisted by one or more peristaltic waves.

Although the main function of swallowing is the propulsion of food from the mouth into the stomach, swallowing also serves as a protective reflex for the upper respiratory tract, preventing unwanted particles from entering the tract. Food or liquid that enters into the airways may act as a locus for infection and this type of infection can be life threatening. For instance, dysphagia after a stroke can be a devastating problem, as it carries a six-fold increased risk of aspiration pneumonia.

Complications that have been associated with dysphagia include pneumonia, malnutrition, dehydration, poorer long-term outcome, increased length of hospital stay, increased rehabilitation time and the need for long-term care assistance, increased mortality, and increased health care costs. These complications impact the physical and social wellbeing of patients, quality of life of both patients and caregivers, and the utilization of health care resources.

In view of the above, there remains a need for improved devices and methods that can treat dysphagia.

US 2019/134389 A1 defines a device for the treatment of dysphagia comprising a flexible member mounting an imaging device for insertion into a patient and one or more electrodes proximate the imaging device for imparting electrical stimulation to the patient.

US 5179952 A discloses a probe for electrocardial stimulation includes a catheterlike conduit for insertion into a patient's esophagus. The conduit has openings which are spaced apart at two longitudinal locations which are closely adjacent to the patient's heart when the probe is positioned in the esophagus. The openings pick up heart sounds and transmit them to the interior of the conduit for communication to the proximal end of the probe, where they can be monitored by attending personnel. Two electrodes are spaced apart longitudinally between and in fixed relation to the openings, so as to position the electrodes accurately relative to the heart based upon heart sounds transmitted through the openings. Other functions, such as a temperature sensor or stomach feeding or pumping capability may also be provided inside the conduit.

### SUMMARY

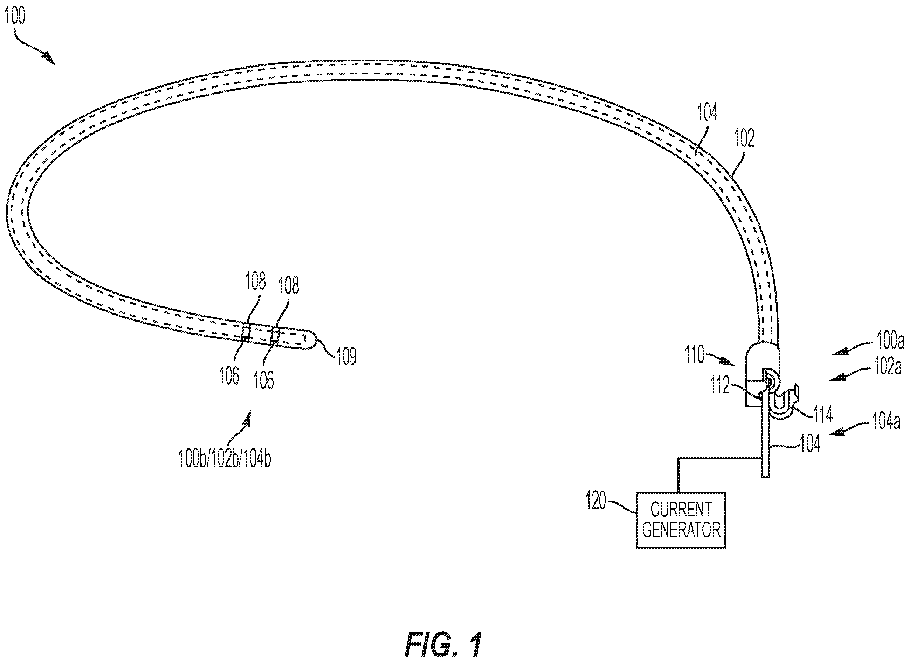

The present technology relates to electrical stimulation devices. According to the invention, the devices are configured to perform pharyngeal electrical stimulation (PES) to treat one or more conditions. Several embodiments of the present disclosure, for example, are configured to perform PES to treat a patient suffering from neurogenic dysphagia. Electrical stimulation of nerves proximate the patient's pharynx increases activity in the motor cortex and other areas of the brain to facilitate a functional reorganization of the centers in the brain responsible for controlling and coordinating swallow function. In some embodiments, an electrical stimulation device can comprise a reusable elongated member having an electrode at its distal portion and a disposable sleeve configured to surround at least a portion of the elongated member when the device is positioned in a patient. The sleeve can be configured to transmit energy from the elongated member to a body lumen of a patient. The subject technology is illustrated, for example, according to various aspects described below, including with reference to FIGS. 1-4.

### BRIEF DESCRIPTION OF THE DRAWINGS

Many aspects of the present disclosure can be better understood with reference to the following drawings. The components in the drawings are not necessarily to scale. Instead, emphasis is placed on illustrating clearly the principles of the present disclosure.
FIG. 1 depicts an electrical stimulation device in accordance with several embodiments of the present technology.
FIG. 2 is a cross-sectional schematic view of the distal portion of the electrical stimulation device shown in FIG. 1.
FIG. 3 depicts an electrical stimulation device transnasally inserted into a pharynx of a patient in accordance with several embodiments of the present technology.
FIG. 4 is a cross-sectional schematic view of a distal portion of an electrical stimulation device configured in accordance with several embodiments of the present technology.

### DETAILED DESCRIPTION

Stimulation of the pharyngeal mucosa with a catheter-based system typically requires the catheter be nasally or orally inserted into the patient's pharynx. To prevent crosscontamination between patients, the catheter can only be used a single time and/or by a single patient. This significantly increases the cost of treatment, as each new patient and/or new treatment requires a new catheter. Moreover, the requirement that the catheter be disposable limits the functionality that can be built into the catheter, as it may be too expensive to include sophisticated functionalities in a disposable medical device.

The devices and systems disclosed herein comprise a reusable component and a disposable component that address the above-noted limitations associated with single-use devices. The present technology may include, for example, a reusable elongated member configured to receive stimulation energy and a sleeve configured to fit over the elongated member. In some embodiments, the elongated member includes one or more first conductive elements at a distal end portion and the sleeve comprises one or more second conductive elements configured to be aligned with and electrically coupled to the first conductive element(s) when the elongated member is inserted into the sleeve. As such, stimulation energy delivered from a current generator to the first conductive element(s) of the elongated member flows to the conductive portion(s) of the sleeve, then to the treatment site to stimulate nearby nerves. In some embodiments, the sleeve comprises a closed distal end such that, when the device is positioned in a body lumen, the sleeve prevents the elongated member from contacting any bodily fluids or tissues, or any other substance in the upper gastrointestinal tract. Thus, while the sleeve is a single-use component, the elongated member can be reused with a new sleeve. Additional details are discussed below with reference to FIGS. 1-4.

FIG. 1 depicts an electrical stimulation device 100 ("device 100") configured in accordance with several embodiments of the present technology. The device 100 has a proximal portion 100a configured to be extracorporeally positioned during treatment, and a distal portion 100b configured to be positioned within a body lumen of a patient (such as a pharynx). As shown in FIG. 1, the device 100 can comprise a sleeve 102 and an elongated member 104 configured to be slidably disposed within a lumen of the sleeve 102. In FIG. 1, the device 100 is shown in an "as assembled" configuration, i.e., with the elongated member 104 inserted into the lumen of the sleeve 102. The sleeve 102 can comprise a proximal portion 102a and a distal portion 102b, and the elongated member 104 can comprise a proximal portion 104a and a distal portion 104b. The device 100 further includes one or more first conductive elements 106 carried by the distal portion 104b of the elongated member 104, and one or more second conductive elements 108 carried by the distal portion 102b of the sleeve 102. The elongated member 104 is configured to be slidably received within the lumen of the sleeve 102 such that the second conductive elements 108 of the sleeve 102 are aligned with and in electrical communication with the first conductive elements 106 of the elongated member 104. The first conductive elements 106 can be configured to mechanically engage the second conductive elements 108 to establish a stable electrical connection between the first and second conductive elements 106, 108. The mechanical engagement can be strong enough to secure the relative positions of the first and second conductive elements 106, 108 during normal use of the device but weak enough to allow a user to separate the sleeve 102 from the elongated member 104 if desired so that the elongated member 104 can be reused when the sleeve 102 is disposed.

As shown in FIG. 1, the sleeve 102 can have a closed distal end 109 such that, when the sleeve 102 is positioned over the elongated member 104, the sleeve 102 fully encapsulates the portion of the elongated member 104 inserted into the patient's body, thus preventing direct contact between the elongated member 104 and the patient's body. This allows the elongated member 104 to be reused, while the sleeve 102 is disposed after treatment.

In some embodiments, the device 100 is configured such that a position of the elongated member 104 can be fixed relative to a position of the sleeve 102 (or vice versa). It may be advantageous to fix the relative positions of the elongated member 104 and the sleeve 102, for instance, to ensure the first and second conductive elements 106, 108 remain longitudinally aligned and in stable electrical communication. For example, as shown in FIG. 1, the proximal portion 100a of the device 100 can include a retaining structure 110 configured to be coupled to the proximal portions of the elongated member 104 and the sleeve 102. The retaining structure 110 can have a first portion 112 on or through which the elongated member 104 and/or sleeve 102 may be movably or fixedly positioned, and a second portion 114 moveable relative to the first portion 112. When the second portion 114 is in an open position (as shown in FIG. 1), the sleeve 102 and the elongated member 104 can move longitudinally relative to one another. When the second portion 114 is in a closed position (not shown), the longitudinal and/or radial positions of the sleeve 102 and the elongated member 104 are substantially fixed relative to one another.

The retaining structure 110 may be fixed to one of the sleeve 102 or the elongated member 104 and, at least in the open configuration, allow movement of the other of the sleeve 102 and the elongated member 104. In some embodiments, for example as shown in FIG. 1, the proximal portion 102a of the sleeve 102 is fixed to the retaining structure 110 and the proximal portion 104a of the elongated member 104 is slidably received by the first portion 112 of the retaining structure 110 when the device 100 is assembled and the second portion 114 is in an open position. When the second portion 114 is in a closed position, the second portion 114 engages the elongated member 104 and fixes the elongated member 104 relative to the sleeve 102 and the retaining structure 110. For example, the second portion 114 can be configured to apply a frictional force to the elongated member 104 if the elongated member 104 is moved longitudinally relative to the sleeve 102 while the second portion 114 is in a closed position. In some embodiments, the second portion 114 is configured to apply a compressive force to the elongated member 104 while the second portion 114 is in a closed position, which can increase a frictional force applied to the elongated member 104 by the second portion 114 that resists longitudinal movement of the elongated member 104.

In some embodiments the retaining structure 110 is a disposable component, and in some embodiments the retaining structure 110 is a reusable component. In any case, the second portion 114 of the retaining structure 110 can comprise a high friction thermoplastic elastomer liner that engages the proximal portion of the elongated member 104 when the second portion 114 is in the closed position. The liner can be configured to reduce the compressive force required to fix the elongated member 104 and thereby prevent pinching of the elongated member 104. Other suitable shapes, materials, and configurations for the retaining structure 110 are possible. For example, the retaining structure 110 can comprise magnets, a screw and threaded insert, a radial compression clip, and/or others to fix the proximal portion of the elongated member 104 to the retaining structure 110 and/or sleeve 102.

In addition to the retaining structure 110 and/or instead of the retaining structure 110, the first and second conductive elements 106, 108 can be configured to engage and/or interlock with one another to facilitate alignment of corresponding first and second conductive elements 106, 108 and/or establishment of sufficient electrical contact between the first and second conductive elements 106, 108. Such engagement and/or interlocking can be releasable such that the elongated member 104 can be separated from the sleeve 102 and reused. Details of such engagement are disclosed herein.

The proximal portion 104a of the elongated member 104 can be configured to be electrically coupled to a current generator 120 for delivering electric current to the one or more first conductive elements 106. In some embodiments, the elongated member 104 is coupled to the current generator 120 via a connector (not shown) at a proximal end of the elongated member 104. The connector can provide an electrical interface between the elongated member 104 and the current generator 120 (or other power source and/or controller). In some embodiments, the connector includes a printed circuit board (PCB) with memory chips including encrypted code. The PCB can store patient and treatment information, and/or any other information required for use of the device 100 with the current generator 120.

The current generator 120 is configured to output medically useful electric current, such as a current for stimulating the nerves proximate the treatment site. The current generator 120, for example, can include a power source and a controller. The controller includes a processor coupled to a memory that stores instructions (e.g., in the form of software, code or program instructions executable by the processor or controller) for causing the power source to deliver electric current according to certain parameters provided by the software, code, etc. The power source of the current generator 120 may include a direct current power supply, an alternating current power supply, and/or a power supply switchable between a direct current and an alternating current. The current generator 120 can include a suitable controller that can be used to control various parameters of the energy output by the power source or generator, such as intensity, amplitude, duration, frequency, duty cycle, and polarity. The current generator 120 may be configured to provide a stimulation energy to the conductive elements 108 that has an intensity, amplitude, duration, frequency, duty cycle, and/or polarity such that the conductive elements 108 apply an electric field at the treatment site that promotes neuroplasticity in the areas of the brain associated with swallowing control. Without being bound by theory, it is believed that the treatment energy of the present technology induces and accelerates a cortical reorganization process whereby responsibility for the control and coordination of swallowing activity is moved from the damaged area of the brain to a complementary area of the cortical centers with intact function. The treatment energy of the present technology may also increase local levels of swallow-related neurotransmitters in the pharynx, such as in the oropharynx. The controller can automatically vary the voltage (to a maximum of 250V) in order to deliver the set current. In some embodiments, the only user adjustable parameter is the stimulation intensity which is derived during treatment level optimization prior to every treatment. Patient specific threshold levels are determined by establishing a sensory threshold followed by measurement of the maximum tolerated stimulation intensity. The controller may automatically calculate the correct stimulation level from the sensory threshold and maximum tolerated stimulation intensity and sets this as the output. For example, the current generator 120 can provide a current of about 1 mA to about 50 mA, about 1 mA to about 40 mA, about 1 mA to about 30 mA, about 1 mA to about 20 mA, or about 1 mA to about 10 mA, at a frequency of about 1 Hz to 50 Hz, 1 Hz to 40 Hz, 1 Hz to 30 Hz, 1 Hz to 20 Hz, 1 Hz to 10 Hz, 2 Hz to 8 Hz, 1 Hz, 2 Hz, 3 Hz, 4 Hz, 5 Hz, 6 Hz, 7 Hz, 8 Hz, 9 Hz, or 10 Hz, and having a pulse width of about 150 µS to 250 µS, 175 µS to 225 µS, or 200 µS.

In some embodiments, instead of or in addition to a controller, the current generator 120 can include drive circuitry. In such embodiments, the current generator 120 can include hardwired circuit elements to provide the desired waveform delivery rather than a software-based generator. The drive circuitry can include, for example, analog circuit elements (e.g., resistors, diodes, switches, etc.) that are configured to cause the power source to deliver electric current according to the desired parameters. For example, the drive circuitry can be configured to cause the power source to deliver periodic waveforms. In some embodiments, the drive circuitry can be configured to cause the power source to deliver a unipolar square wave.

The current generator 120 may also be configured to monitor contact quality between the second conductive elements 108 and patient tissue during treatment set up and/or optimization and throughout the treatment process. In some embodiments, the current generator 120 records and stores patient information and includes a USB port to enable downloading of patient data. The current generator may include a touch screen user interface and software to guide a user through the treatment process.

As the elongated member 104 is configured to be reusable between treatment sessions and/or patients, the elongated member 104 can include more sophisticated, and therefore more expensive, functionalities. For example, in some embodiments the elongated member 104 comprises one or more imaging devices that enable a user of the device 100 to visualize the patient's body lumen and/or a portion of the device 100. The imaging device can be located at any location along the length of the elongated member 104 configured to be inserted into the body. For example, the imaging device may be positioned at a location proximal to the conductive elements, between the conductive elements, or distal of the conductive elements, including at a distal terminus of the elongated member 104. In some embodiments, the imaging device is located at a position along the elongated member 104 that allows a user to visualize the conductive elements and/or the treatment site, for example to determine if the electrodes are in contact with tissues of the body lumen at the treatment site. In such embodiments, the sleeve 102 can comprise an optically transparent portion configured to be aligned with the imaging device when the elongated member 104 is inserted into the sleeve 102.

Additionally or alternatively, in some embodiments the elongated member 104 comprises one or more articulatable elements configured to provide positional control of the device 100 to improve contact between the second conductive elements 108 and the tissues of the body lumen at the treatment site and/or reduce patient discomfort and trauma to the body lumen during insertion of the device 100. One such articulatable element may comprise a distal tip configured to be deflected downwardly upon contact of the tip with a posterior wall of a nasal cavity of the patient, thus making it easier to continue the insertion process and feed the catheter into a nasopharynx of the patient and beyond. In some embodiments, the articulatable element is configured to urge the second conductive elements 108 into contact with the posterior wall of an oropharyngeal region of the patient to improve contact with target tissues and the nerves therein. In some embodiments, the articulatable element comprises a hinge feature at the external entrance to the patient's nostrils to reduce pressure on patient tissues whilst urging the device 100 along the base of the nasal cavity.

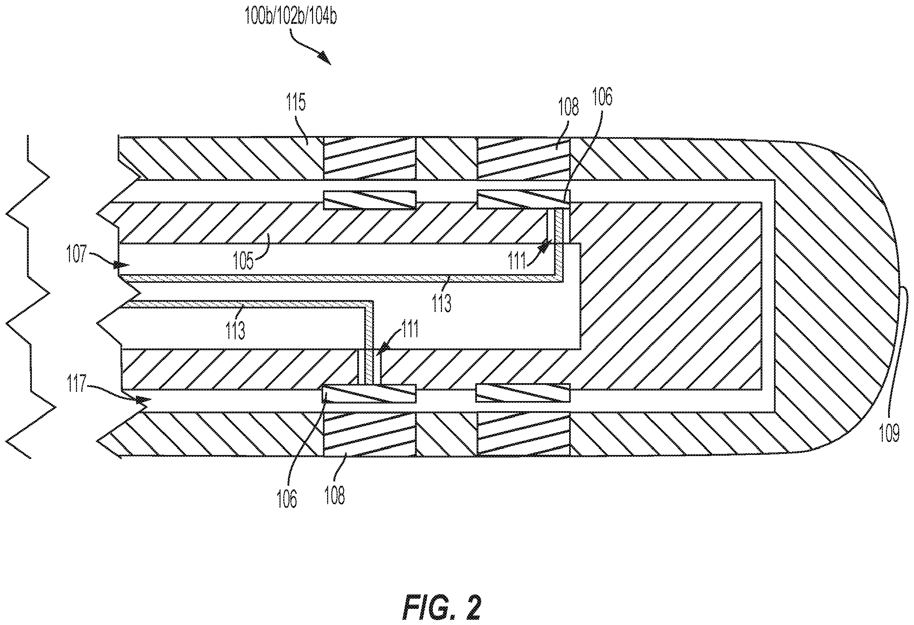

FIG. 2 is a cross-sectional view of the distal portion 100b of the device 100 shown in FIG. 1. In some embodiments, the elongated member 104 includes a sidewall 105 extending between the proximal and distal portions 104a, 104b and a channel 107 defined by the sidewall 105 and extending from the proximal portion 104a towards the distal portion 104b. As shown in FIG. 2, the first conductive elements 106 of the elongated member 104 can be spaced apart from one another and/or from a distal terminus of the elongated member 104. In these and other embodiments, one or both of the first conductive elements 106 can extend around the full circumference of the elongated member 104. In some embodiments, one of the first conductive elements 106 comprises the distal terminus of the elongated member 104. In some embodiments, one, some, or all of the conductive elements extend around only a portion of a circumference of the elongated member 104. For example, one or both of the first conductive elements 106 may not extend around the full circumference of the elongated member 104. While the embodiments depicted in FIGS. 1 and 2 include two first conductive elements 106, in some embodiments the elongated member 104 may include a single conductive element configured to deliver stimulation energy in a monopolar fashion, or may include three or more conductive elements spaced apart along the longitudinal axis of the elongated member 104 and/or spaced apart around a circumference of the elongated member 104. A spacing of the first conductive elements 106 with respect to one another can be selected based on one or more desired properties (e.g., current density, depth, etc.) of the electric field to be generated by the device 100. Moreover, a spacing of the first conductive elements 106 with respect to the distal terminus of the elongated member 104 can be selected based on the location of the treatment site within the patient's body lumen to be stimulated. For example, the first conductive elements 106 can be spaced apart from the distal terminus of the elongated member 104 to facilitate contact of the first conductive elements 106 with an oropharyngeal region of the patient's pharynx (via the second conductive elements 108).

Each of the first conductive elements 106 may comprise an electrical connector, an electrode, an exposed portion of a conductive material, a printed conductive material, and/or other suitable forms. In some embodiments, for example as shown in FIG. 2, each of the first conductive elements 106 comprises a ring electrode and together are configured to deliver bipolar stimulation energy. The electrode can be crimped, welded, or otherwise adhered to an outer surface of the sidewall 105. In some embodiments, the first conductive elements 106 comprise a flexible conductive material disposed on the elongated member 104 via printing, thin film deposition, or other suitable techniques.

The elongated member 104 may further include a conducting element 113, such as one or more leads, for every first conductive element 106. In some embodiments, the one or more conducting elements 113 are positioned within and extend along the channel 107 of the elongated member 104. In some embodiments, each of the conducting elements 113 extends along a separate channel within the elongated member 104. Such separate channels can be located at distinct radial positions. Each conducting element 113 can have a proximal end configured to be coupled to an electric current generator (such as current generator 120) and a distal end configured to be coupled to one of the first conductive elements 106. According to various embodiments, the distal end of the conducting element 113 can comprise the corresponding first conductive element 106. In some embodiments, the conducting elements 113 are electrically coupled to a connector (not shown) at the proximal portion 100a of the device 100. In some embodiments, each of the conducting elements 113 extends through an aperture 111 in the sidewall 105 to electrically couple to a corresponding one of the first conductive elements 106, a corresponding one of the second conductive elements 108, and/or a connector coupled to the corresponding one of the second conductive elements 108. In some embodiments, the conducting elements 113 are insulated along all or a portion of their respective lengths.

Referring still to FIG. 2, the sleeve 102 can comprise a sidewall 115 extending between the proximal and distal portions 102a, 102b and a lumen 117 defined by the sidewall 115 and extending from the proximal portion 102a towards the closed distal end 109. As previously mentioned, the distal end 109 of the sleeve 102 is closed and thus prevents ingress of bodily fluids or tissues into the lumen 117 when the device 100 is positioned within a body lumen of a patient. Accordingly, the sleeve 102 is configured to prevent the elongated member 104 from contacting bodily fluids or tissues so that the elongated member 104 can be reused between patients and/or between treatment sessions. The closed end 109 of the sleeve 102 may be rounded, for example, for patient comfort and for ease of insertion into the patient.

The second conductive elements 108 of the sleeve 102 can be spaced apart from one another and/or from a distal terminus of the sleeve 102. In these and other embodiments, one or both of the second conductive elements 108 can extend around the full circumference of the sleeve 102. In some embodiments, one of the second conductive elements 108 comprises the distal terminus of the sleeve 102. In some embodiments, one, some, or all of the second conductive elements 108 extend around only a portion of a circumference of the sleeve 102. While the embodiments depicted in FIGS. 1 and 2 include two second conductive elements 108, in some embodiments the sleeve 102 may include a single conductive element configured to deliver stimulation energy in a monopolar fashion, or may include three or more conductive elements spaced apart along the longitudinal axis of the sleeve 102. The device 100 may include the same number or a different number of first conductive elements 106 and second conductive elements 108.

Each of the second conductive elements 108 may comprise an electrode, an exposed portion of a conductive material, a printed conductive material, and other suitable forms. In some embodiments, for example as shown in FIG. 2, the second conductive elements 108 each comprise a ring electrode and together are configured to deliver bipolar stimulation energy. The electrode can be crimped, welded, or otherwise adhered to an outer surface of the sidewall 115. In some embodiments, the second conductive elements 108 comprise a flexible conductive material disposed on the sleeve 102 via printing, thin film deposition, or other suitable techniques.

Although FIG. 2 depicts a gap between an outer surface of the elongated member 104 and an inner surface of the sleeve 102, in some embodiments the lumen 117 of the sleeve 102 is sized such that, when the elongated member 104 is positioned within the lumen 117, each of the first conductive elements 106 contact a corresponding one of the second conductive elements 108 so that the first conductive elements 106 and the second conductive elements 108 are in electrical communication. In some embodiments, the device 100 can comprise one or more clamps, clips, magnets, ties, or other fasteners configured to cause the first and second conductive elements 106, 108 to contact one another. As but one example, a clamp can be carried by the sleeve 102 at a location near the first and second conductive elements 106, 108 (e.g., distal of the retaining structure 110, at or adjacent to a distal end of the device 100, etc.). The clamp can be movable between an open position and a closed position. When the clamp is in the open position, there may be a radial gap between an outer surface of the elongated member 104 and a luminal surface of the sleeve 102 such that the elongated member 104 can be slidably moved within the lumen 117 of the sleeve 102. When the clamp is in the closed position, the clamp can apply a radially inwardly directed force to the device 100 such that the radial gap between the outer surface of the elongated member 104 and the luminal surface of the sleeve 102 is reduced or eliminated, thereby causing the first and second conductive elements 106, 108 to contact one another. In some embodiments, the elongated member 104 can comprise an articulatable element configured to cause the first conductive elements 106 to move radially outwardly and into contact with the second conductive elements 108. Non-exhaustive examples of such articulatable elements can comprise a balloon, a crankshaft, a turnbuckle, a spring, etc.

Each aligned pair of a first conductive element 106 and a second conductive element 108 may comprise a conductor. For example, a proximal first conductive element 106 and a proximal second conductive element 108 may be aligned to comprise a proximal conductor and a distal first conductive element 106 and a distal second conductive element 108 may be aligned to comprise a distal conductor. As used herein to describe the relative positions of the conductive elements, "align" refers to a position in which all or a portion of a first conductive element 106 is longitudinally coextensive with all or a portion of a second conductive element 108.

The spacing between the proximal and distal conductors along the distal portion of the elongated member 104 affects the current density and the shape of the electric field and thus the effectiveness of the delivered stimulation energy. For example, the greater the distance between the proximal and distal conductors, the deeper the electric field generated by the proximal and distal conductors will penetrate into the patient tissue and the lower the current density per unit area. Likewise, the greater the distance between the proximal and distal conductors, the less the patient will feel or detect the electrical stimulation at a given (fixed) current level. Conversely, if the conductive elements 106 are positioned closer together, the resulting electric field will be smaller (i.e., will not penetrate as deeply into the tissue) and the current density will be higher, meaning the patient will feel more sensation/discomfort at a given (fixed) current level.

The location of the proximal and distal conductors along the distal portion of the elongated member 104 also affects the effectiveness of the delivered stimulation energy in that it limits the practical use of the device. For example, whether the conductors are at the terminus of the elongated member 104 or located on the side of the elongated member 104 affects the construction of at least the distal portion of the elongated member 104 and thus the stiffness of the elongated member 104 at the distal portion. This, in turn, affects the ability to manipulate the insertion path of the elongated member 104 and/or the orientation of the elongated member 104 at the treatment site, which affects whether the elongated member 104 can position the conductors in sufficient contact with the targeted tissue (such as the posterior wall of the pharynx) and/or maintain that contact. For example, locating the conductors at the distal terminus might allow for a more flexible and/or manipulatable distal portion, thus enabling the elongated member 104 to be moved to different areas of the pharyngeal and oral spaces. It would be difficult, however, to ensure sufficient contact between a terminally-located conductor and certain anatomical regions generally parallel with the longitudinal axis of the inserted elongated member 104 (such as the posterior pharyngeal wall). Positioning the conductors along the sidewall of the elongated member 104 and spaced apart from each other and the distal terminus, however, ensures better contact with body tissue parallel to the longitudinal axis of the device 100 (such as the posterior pharyngeal wall) when inserted, and also provides electric field coverage at a therapeutic current density within a treatment zone containing the targeted nerves. The treatment zone, for example, may encompass an area proximate the posterior pharyngeal wall containing the targeted nerves.

Any of the elongated members and/or sleeves disclosed herein may be formed of a flexible material such as a thermoplastic elastomer (e.g., Pebax^{®}), polyurethane, or another material suitable for forming devices. In some embodiments, the elongated member and/or sleeve comprises a transparent material and/or includes one or more transparent portions. In some embodiments, the elongated member and/or sleeve is formed of multiple layers of material. For example, the elongated member and/or sleeve can comprise an outer layer of a flexible material (e.g., polyurethane) and an inner layer of a material with a low coefficient of friction and/or high dielectric strength (e.g., fluorinated ethylene propylene). An elongated member of the present technology may comprise a structure such as, but not limited to, wire, a coil, or a braid, within a sidewall of the elongated member for reinforcement and/or kink-resistance. In some embodiments, the elongated member and/or the sleeve comprise a pre-set shape configured to facilitate insertion of the device and/or to urge the conductive elements of the sleeve into contact with the patient's pharynx. Although FIG. 2 schematically depicts the elongated member comprising a substantially planar distal end, the elongated member and/or the sleeve can comprise a substantially rounded distal end. In some embodiments, the distal end of the sleeve can be tapered such that a stiffness of the distal end of the sleeve is less than a stiffness of the other portions of the sleeve.

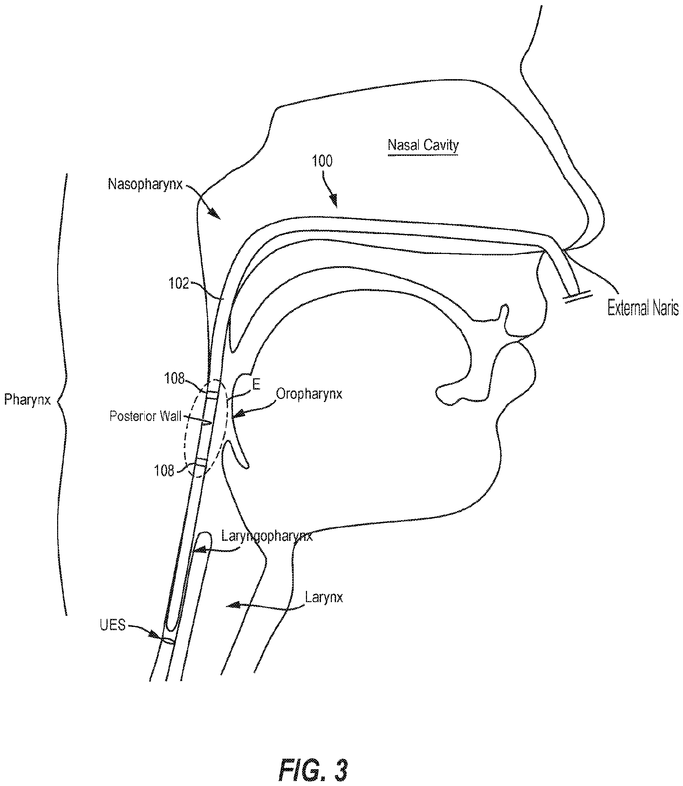

For a given treatment session, the elongated member 104 can be inserted into the sleeve 102 (or the sleeve 102 advanced over the elongated member 104) and the relative positions of the sleeve 102 and elongated member 104 are fixed prior to inserting the device 100 into the patient. The device 100 can be nasally or orally inserted into the patient's body until the distal portion 100b of the device 100 is positioned proximate a treatment site within the patient's pharynx. For example, as shown in FIG. 3, the device 100 can be inserted through a nostril of a patient and advanced distally through the nasal cavity and nasopharynx until the second conductive elements 108 at the distal portion 100b of the device 100 are positioned in apposition with a pharyngeal wall, such as an oropharyngeal region of the wall. In some embodiments, the second conductive elements 108 are positioned in apposition with a posterior portion of the pharyngeal wall. According to several embodiments, one or both of the second conductive elements 108 may be aligned with another portion of the pharynx, such as the nasopharynx and/or the laryngopharynx. In these and other embodiments, the second conductive elements 108 are positioned within the pharynx (any of the nasopharynx, oropharynx, and laryngopharynx) in contact with a portion of the pharyngeal wall opposite the C3/C4 vertebral junction.

In some embodiments, the sleeve 102 and/or the elongated member 104 can comprise one or more indicators configured to facilitate insertion and positioning of the device 100 within the patient. For example, the indicator can comprise one or more visual markings that, when viewed via the patient's oral cavity, indicate the device 100 is properly positioned or that the device 100 should be inserted further or withdrawn. In some embodiments, the indicator comprises one or more circumferential markings (such as one or more colored bands) printed on the sleeve 102 and/or the elongated member 104.

When the distal portion 100b of the device 100 is in a desired position, stimulation energy is delivered from the first conductive elements 106 to the treatment site via the second conductive elements 108. In some embodiments, the proximal conductor (e.g., the proximal pair of first and second conductive elements 106, 108) is configured to be negatively charged and the distal conductor (e.g., the distal pair of first and second conductive elements 106, 108) are configured to be positively charged (or vice versa). Accordingly, when current is supplied to the elongated member 104 (e.g., via the conducting elements 113), current flows radially away from the proximal pair of conductive elements to the tissues and nerves at or radially adjacent the patient's pharynx, then turns and flows radially toward the distal pair of conductive elements. As shown in FIG. 3, the resulting electric field E generally takes the shape of an oblate spheroid, where the depth at which the electric field E extends away from the device 100 is based, at least in part, on a spacing between the conductive elements 108 and/or on a magnitude of the current supplied to the elongated member 104. In some embodiments, the delivered current is a unipolar square wave having an amplitude between 1 mA and 50 mA, a frequency of 5 Hz, and a pulse duration of 200 µS. Each treatment session can have a duration between 5 minutes and 20 minutes. For example, the treatment session can have a duration of 10 minutes. In some embodiments, a patient can undergo a single treatment per day over the course of multiple days of treatment. For example, a patient can undergo one treatment session per day for three to six consecutive days. In some embodiments, the patient may undergo multiple treatment sessions per day and/or per week. Still, other current parameters and treatment parameters are possible.

In any case, when a given treatment session is completed, the sleeve 102 can be disposed while the elongated member 104 can be re-used with a new sleeve 102 for a new treatment session with the same patient, or for a new treatment session with a different patient.

The devices of the present technology are subjected to myriad forces during insertion and use (e.g., stretching, twisting, bending, compression, tension, shearing, etc.). As but one example, the device may bend one or more times as the device is navigated through the patient's nasal cavity and into the patient's pharynx. Motion and deformation of the device can cause the elongated member and sleeve to move relative to one another, which can cause the corresponding first and second conductive elements to move relative to one another. If the relative movement between the first and second conductive elements is too great, the conductive elements may no longer be in sufficient electrical contact. To facilitate establishment and maintenance of sufficient electrical contact between the first and second conductive elements, the devices of the present technology comprise first and second conductive elements configured to temporarily engage and/or interlock with one another.

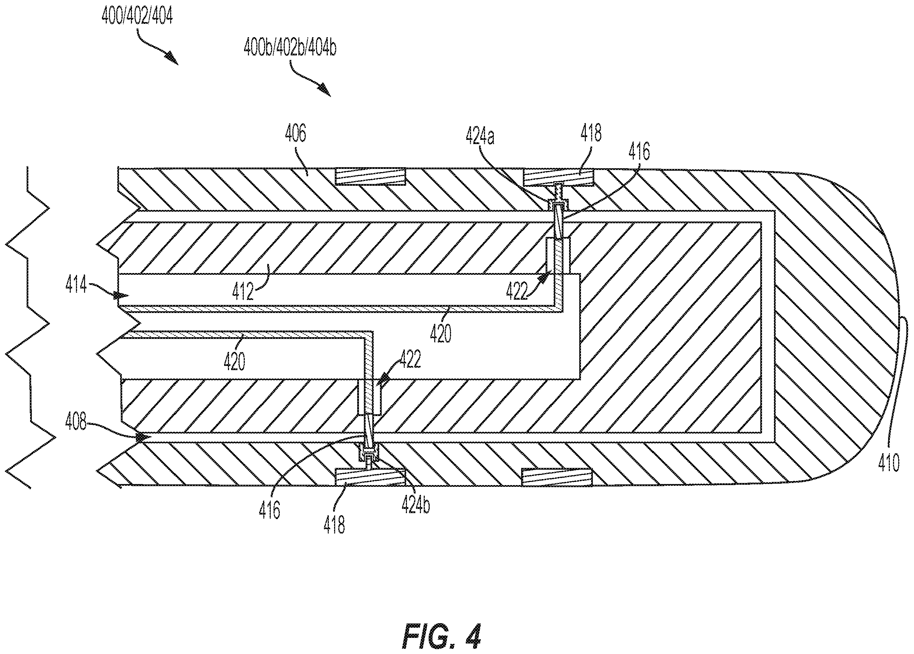

For example, FIG. 4 is a cross-sectional view of a distal portion 400b of a device 400 having conductive elements with one or more interlocking features. The device 400 can be similar to any of the devices disclosed herein (e.g., device 100), except as described below. For example, the device 400 can comprise an elongated member 404 and a sleeve 402 configured to be positioned over at least a portion of the elongated member 404. The sleeve 402 can comprise a sidewall 406 defining a lumen 408. In various embodiments, the lumen 408 does not extend through a distal end 410 of the sleeve 402 such that the distal end 410 is closed, which can prevent ingress of bodily fluids or tissues into the lumen 408 when the device 400 is positioned within a body lumen of a patient. The closed end 410 of the sleeve 402 may be rounded, for example, for patient comfort and for ease of insertion into the patient. The elongated member 404 can be configured to be at least partially positioned within the lumen 408 of the sleeve 402. The elongated member 404 can comprise a sidewall 412 defining one or more channels 414.

As shown in FIG. 4, the elongated member 404 can carry one or more first conductive elements 416 and the sleeve 402 can carry one or more second conductive elements 418. The elongated member 404 can carry one or more conducting elements 420, such as leads, wires, cables, etc., positioned within and extending along the one or more channels 414 and having a proximal end configured to be electrically coupled to an electric current generator and a distal end coupled to and/or comprising one or more of the first conductive elements 416. In some embodiments, the elongated member 404 includes one or more apertures 422 extending through the thickness of its sidewall 412 at its distal portion 404b, and each of the conducting elements 420 extend through a corresponding one of the apertures 422. In some embodiments, the conducting elements 420 are insulated along all or a portion of their respective lengths.

The first conductive elements 416 of the elongated member 404 can be spaced apart from one another and/or from a distal terminus of the elongated member 404. One, some, or all the first conductive elements 416 can extend partially or fully around the circumference of the elongated member 404. In some embodiments, one or more of the first conductive elements 416 comprises the distal terminus of the elongated member 404. While the embodiment depicted in FIG. 4 includes two first conductive elements 416, in some embodiments the elongated member 404 may include a single first conductive elements 416 configured to deliver stimulation energy in a monopolar fashion, or may include three or more first conductive elements 416 spaced apart along the longitudinal axis of the elongated member 404 and/or spaced apart around a circumference of the elongated member 404. A spacing of the first conductive elements 416 with respect to one another can be selected based on one or more desired properties (e.g., current density, depth, etc.) of the electric field to be generated by the device 400. Moreover, a spacing of the first conductive elements 416 with respect to the distal terminus of the elongated member 404 can be selected based on the location of the treatment site within the patient's body lumen to be stimulated. For example, the first conductive elements 416 can be spaced apart from the distal terminus of the elongated member 404 to facilitate contact of the first conductive elements 416 with an oropharyngeal region of the patient's pharynx (via second conductive elements of the sleeve 402).

The second conductive elements 418 of the sleeve 402 can be spaced apart from one another and/or from a distal terminus of the sleeve 402. In these and other embodiments, one, some, or all of the second conductive elements 418 can extend partially or fully around the circumference of the sleeve 402. In some embodiments, one or more of the second conductive elements 418 comprises the distal terminus of the sleeve 402. While the embodiment depicted in FIG. 4 includes two second conductive elements 418, in some embodiments the sleeve 402 may include a single second conductive element 418 configured to deliver stimulation energy in a monopolar fashion, or may include three or more second conductive elements 418 spaced apart along the longitudinal axis of the sleeve 402. The device 400 may include the same number or a different number of first conductive elements 416 and second conductive elements 418.

In some embodiments, one, some, or all of the second conductive elements 418 comprise a conductive material. For example, any of the second conductive elements 418 can comprise an electrode, an exposed portion of a conductive material, a printed conductive material, and/or other suitable forms. In some embodiments, the second conductive elements 418 each comprise a ring electrode and together are configured to deliver bipolar stimulation energy. The second conductive elements 418 can be crimped, soldered, welded, glued, or otherwise secured to an outer surface of the sidewall 406 of the sleeve 402. In some embodiments, the second conductive elements 418 comprise a conductive material disposed on and/or in the sidewall 406 of the sleeve 402. Such conductive material can be positioned on and/or in the sidewall 406 via printing, thin film deposition, coating, extrusion, or other suitable techniques. In some embodiments, one, some, or all of the second conductive elements 418 are flexible.

In some embodiments, one, some, or all of the first conductive elements 416 comprise a conductive material. The first conductive elements 416 can be electrically coupled to the conducting elements 420 to receive energy from an electric current generator via the conducting elements 420. In some embodiments, the distal ends of the conducting elements 420 comprise the first conductive elements 416. The first conductive elements 416 can be configured to electrically couple with the second conductive elements 418 (either directly or indirectly) such that energy can be transferred between from the conducting elements 420 to the first conductive elements 416, from the first conductive elements 416 to the second conductive elements 418, and from the second conductive elements 418 to the patient's body lumen and surrounding tissues.

Electrically coupling corresponding first and second conductive elements 416, 418 can comprise at least partially aligning the first and second conductive elements 416, 418 along a longitudinal dimension of the device 400. In some embodiments, electrically coupling corresponding first and second conductive elements 416, 418 comprises causing the first and second conductive elements 416, 418 to contact. The transfer of energy from the first conductive elements 416 to the second conductive elements 418 can at least partially depend on an area of contact between the first and second conductive elements 416, 418. In some embodiments, the first and second conductive elements 416, 418 are mechanically secured and/or interlocked to one another (either directly or indirectly). The device 400 can be subject to mechanical forces during insertion and/or use that may inadvertently modify alignment of and/or contact between corresponding first and second conductive elements 416, 418, which can degrade a quality of the electrical connection between the first and second conductive elements 416, 418. Mechanically securing and/or interlocking the first and second conductive elements 416, 418 can prevent or limit such misalignment and/or disengagement of the first and second conductive elements 416, 418 during insertion and/or use of the device 400. In some embodiments, it may be desirable for the mechanical securing and/or interlocking of the corresponding first and second conductive elements 416, 418 to be selectively releasable such that, after use of the device 400, the sleeve 402 and the elongated member 404 can be separated and the elongated member 404 can be reused.

The first and second conductive elements 416, 418 can be coupled either directly or indirectly. For example, as shown in FIG. 4, the device 400 can comprise one or more connectors 424 configured to electrically couple to one of the first conductive elements 416 (either directly or indirectly) and configured to electrically couple to one of the second conductive elements 418 (either directly or indirectly). In some embodiments, the connectors 424 are carried by the sleeve 402 (see FIG. 4, for example). Additionally or alternatively, one, some, or all of the connectors 424 can be carried by the elongated member 404. One, some, or all of the connectors 424 can be distinct from the first and second conductive elements 416, 418 or can be continuous and monolithic with one of the first or second conductive elements 416, 418. In some embodiments, the device 400 does not include the connectors 424 and the first and second conductive elements 416, 418 are directly coupled to (e.g., in direct contact with) one another.

The connectors 424 can comprise any suitable electrical connector including, male connectors, female connectors, and/or genderless connectors. In some embodiments, the first conductive element 416 and/or the second conductive element 418 has a gender that is opposite to the gender of the portion of the connector 424 that conductive element is configured to be coupled to. For example, FIG. 4 depicts a first connector 424a having a female connector portion configured to engage the corresponding first conductive element 416 and a male connector portion configured to engage the corresponding second conductive element 418. FIG. 4 also depicts a second connector 424b having two female connector portions each configured to engage male connector portions of the respective first and second conductive elements 416, 418. Female connectors and/or female connector portions disclosed herein can comprise sockets, recesses, openings, receptacles, indentations, terminals, combinations thereof, and/or others. Male connectors and/or male connector portions disclosed herein (e.g., the male connector portion of the first connector 424a, the male connector portions of the first and second conductive elements 416, 418, etc.) can comprise pins, protrusions, bumps, clips, wires, combinations thereof, and/or others.

Electrically coupling the first and second conductive elements 416, 418 via male and female connectors (directly or indirectly) can facilitate establishing and maintaining stable electrical contact between the first and second conductive elements 416, 418. For example, because a female connector limits motion of a male connector that is inserted in the female connector, the male connector can be accurately located at a desired position. As a result, the alignment of corresponding first and second conductive elements 416, 418 can be accurately controlled via coupling of the male and female connectors. Moreover, because a female connector at least partially surrounds a male connector inserted therein, an area of contact between the connectors can be greater than an area of contact between planar elements. In some embodiments, male and female connectors mechanically interlock (e.g., via interference fit, snap fit, etc.), which can prevent or limit separation of and reduction in electrical contact between the first and second conductive elements 416, 418.

In some embodiments, one or more second conductive elements 418 can comprise a ring electrode positioned on an outer surface of the sleeve 402 and electrically coupled to a connector positioned within the sidewall 406 of the sleeve 402. Such connector can comprise a conductive material in the form of a wire, a conductive ink, a conductive film, a pin, etc. In some embodiments, the second conductive element 418 is electrically coupled to the connector via a permanent connection (e.g., a soldered connection, a crimped connection, etc.) or a releasable connection (e.g., pin and socket, screw terminal, etc.). The connector positioned within the sidewall 406 of the sleeve 402 can also be configured to be electrically coupled to a corresponding first conductive element 416 of the elongated member 404.

According to various embodiments, corresponding first and second conductive elements 416, 418 can be configured to be directly electrically coupled in a manner that facilitates aligning and engaging of and/or maintains alignment and engagement of the first and second conductive elements 416, 418. For example, the first and second conductive elements 416, 418 can produce magnetic fields of opposing polarities such that the corresponding first and second conductive elements 416, 418 are magnetically attracted to one another. The attractive magnetic forces acting on the first and second conductive elements 416, 418 can be sufficiently large to prevent the first and second conductive elements 416, 418 from becoming misaligned and/or poorly engaged during insertion and/or use of the device 400.

### Conclusion

Although many of the embodiments are described above with respect to systems, devices, and methods, which do not form part of the invention, for electrically stimulating a pharynx of a patient to treat a patient suffering from dysphagia, the technology is applicable to other applications and/or other approaches.

The descriptions of embodiments of the technology are not intended to be exhaustive or to limit the technology to the precise form disclosed above. Where the context permits, singular or plural terms may also include the plural or singular term, respectively. Although specific embodiments of, and examples for, the technology are described above for illustrative purposes, various equivalent modifications are possible within the scope of the technology, as those skilled in the relevant art will recognize. For example, while steps are presented in a given order, alternative embodiments may perform steps in a different order. The various embodiments described herein may also be combined to provide further embodiments.

Moreover, unless the word "or" is expressly limited to mean only a single item exclusive from the other items in reference to a list of two or more items, then the use of " or" in such a list is to be interpreted as including (a) any single item in the list, (b) all of the items in the list, or (c) any combination of the items in the list. Additionally, the term "comprising" is used throughout to mean including at least the recited feature(s) such that any greater number of the same feature and/or additional types of other features are not precluded. It will also be appreciated that specific embodiments have been described herein for purposes of illustration, but that various modifications may be made without deviating from the technology. Further, while advantages associated with certain embodiments of the technology have been described in the context of those embodiments, other embodiments may also exhibit such advantages, and not all embodiments need necessarily exhibit such advantages to fall within the scope of the technology. Accordingly, the disclosure and associated technology can encompass other embodiments not expressly shown or described herein.

As used herein, the terms "generally," "substantially," "about," and similar terms are used as terms of approximation and not as terms of degree, and are intended to account for the inherent variations in measured or calculated values that would be recognized by those of ordinary skill in the art.

## Claims

1. A device (100) comprising:
a sleeve (102) configured to be inserted into a pharynx of a patient, the sleeve having a proximal end portion (102a), a distal end portion (102b), a lumen (117) extending from the proximal end portion to the distal end portion, and a conductive portion (108) spaced apart from the distal end portion (102b) of the sleeve (102); and
an elongated member (104) having an electrode (106) positioned at an outer surface of the elongated member,
**characterized in that**
the elongated member (104) is configured to be positioned within the lumen (117) of the sleeve (102) such that the electrode (106) is in electrical communication with the conductive portion (108) of the sleeve (102), and
wherein the device (100) is configured to deliver energy from the electrode (106) to the pharynx via the conductive portion (108) of the sleeve (102).

2. The device (100) of Claim 1, wherein the device is configured to electrically stimulate nerves proximate an oropharyngeal region of the pharynx to treat dysphagia.

3. The device (100) of any one of the preceding Claims, wherein, when the elongated member (104) is positioned within the lumen (117) of the sleeve (102) and the device (100) is inserted into the pharynx of the patient, the sleeve (102) prevents direct contact between the elongated member (104) and the patient's body and contents thereof.

4. The device (100) of any one of the preceding Claims, wherein the conductive portion (108) comprises a ring electrode disposed on an outer surface of the sleeve (102).

5. The device (100) of any one of the preceding Claims, wherein the electrode (106) is configured to mechanically contact the conductive portion (108) when the elongated member (104) is positioned within the lumen (117) of the sleeve (102).

6. The device (100) of any one of the preceding Claims, wherein the sleeve (102) is disposable and the elongated member (104) is reusable.

7. The device (100) of any one of the preceding Claims, wherein the sleeve (102) has a closed distal end (109).

8. The device (100) of any one of the preceding Claims, wherein the elongated member (104) includes a conducting element (113) having a distal end portion electrically coupled to the electrode (106) and a proximal end portion configured to be coupled to a current generator.

9. The device (100) of Claim 8, wherein the conducting element (113) comprises a wire insulated along at least a portion of a length of the wire.

10. The device (100) of Claim 8 or 9, wherein the conducting element (113) is positioned within a channel (107) extending through the elongated member (104).

11. The device (100) of any one of the preceding Claims, further comprising a retaining structure (110) configured to releasably fix a position of the sleeve (102) and the elongated member (104) relative to one another.

12. The device (100) of any one of the preceding Claims, wherein the device is configured to be nasally or orally inserted into the pharynx of the patient.

13. The device (100) of any one of the preceding Claims, wherein the elongated member (104) includes a camera at its distal end portion (102a).

14. The device (100) of any one of the preceding Claims, wherein the elongated member (104) includes an articulatable element.

15. The device (100) of any one of the preceding Claims, wherein the conductive portion (108) comprises a flexible printed conductive material.

## Patentansprüche

1. Vorrichtung (100), umfassend:
eine Hülse (102), die konfiguriert ist, um in den Rachen eines Patienten eingeführt zu werden, wobei die Hülse einen proximalen Endabschnitt (102a), einen distalen Endabschnitt (102b), ein Lumen (117), das sich von dem proximalen Endabschnitt zu dem distalen Endabschnitt erstreckt, und einen leitenden Abschnitt (108), der von dem distalen Endabschnitt (102b) der Hülse (102) beabstandet ist, aufweist; und
ein längliches Element (104) mit einer Elektrode (106), die an einer Außenfläche des länglichen Elements positioniert ist,
**dadurch gekennzeichnet, dass**
das längliche Element (104) konfiguriert ist, um innerhalb des Lumens (117) der Hülse (102) positioniert zu sein, sodass die Elektrode (106) in elektrischer Kommunikation mit dem leitenden Abschnitt (108) der Hülse (102) ist, und
wobei die Vorrichtung (100) konfiguriert ist, um Energie von der Elektrode (106) an den Rachen über den leitenden Abschnitt (108) der Hülse (102) abzugeben.

2. Vorrichtung (100) nach Anspruch 1, wobei die Vorrichtung konfiguriert ist, um Nerven nahe einem oropharyngealen Bereich des Rachens elektrisch zu stimulieren, um Dysphagie zu behandeln.

3. Vorrichtung (100) nach einem der vorhergehenden Ansprüche, wobei, wenn das längliche Element (104) innerhalb des Lumens (117) der Hülse (102) positioniert ist und die Vorrichtung (100) in den Rachen des Patienten eingeführt ist, die Hülse (102) direkten Kontakt zwischen dem länglichen Element (104) und dem Körper des Patienten und Inhalten davon verhindert.

4. Vorrichtung (100) nach einem der vorhergehenden Ansprüche, wobei der leitende Abschnitt (108) eine Ringelektrode umfasst, die auf einer Außenfläche der Hülse (102) angeordnet ist.

5. Vorrichtung (100) nach einem der vorhergehenden Ansprüche, wobei die Elektrode (106) konfiguriert ist, um den leitenden Abschnitt (108) mechanisch zu kontaktieren, wenn das längliche Element (104) innerhalb des Lumens (117) der Hülse (102) positioniert ist.

6. Vorrichtung (100) nach einem der vorhergehenden Ansprüche, wobei die Hülse (102) wegwerfbar ist und das längliche Element (104) wiederverwendbar ist.

7. Vorrichtung (100) nach einem der vorhergehenden Ansprüche, wobei die Hülse (102) ein geschlossenes distales Ende (109) aufweist.

8. Vorrichtung (100) nach einem der vorhergehenden Ansprüche, wobei das längliche Element (104) ein leitendes Element (113) mit einem distalen Endabschnitt, der elektrisch an die Elektrode (106) gekoppelt ist, und einem proximalen Endabschnitt, der konfiguriert ist, um an einen Stromgenerator gekoppelt zu sein, beinhaltet.

9. Vorrichtung (100) nach Anspruch 8, wobei das leitende Element (113) einen Draht umfasst, der entlang zumindest eines Abschnittes einer Länge des Drahtes isoliert ist.

10. Vorrichtung (100) nach Anspruch 8 oder 9, wobei das leitende Element (113) innerhalb eines Kanals (107) positioniert ist, der sich durch das längliche Element (104) erstreckt.

11. Vorrichtung (100) nach einem der vorhergehenden Ansprüche, ferner umfassend eine Haltestruktur (110), die konfiguriert ist, um eine Position der Hülse (102) und des länglichen Elements (104) relativ zueinander lösbar zu fixieren.

12. Vorrichtung (100) nach einem der vorhergehenden Ansprüche, wobei die Vorrichtung konfiguriert ist, um nasal oder oral in den Rachen des Patienten eingeführt zu werden.

13. Vorrichtung (100) nach einem der vorhergehenden Ansprüche, wobei das längliche Element (104) eine Kamera an seinem distalen Endabschnitt (102a) beinhaltet.

14. Vorrichtung (100) nach einem der vorhergehenden Ansprüche, wobei das längliche Element (104) ein gelenkiges Element beinhaltet.

15. Vorrichtung (100) nach einem der vorhergehenden Ansprüche, wobei der leitende Abschnitt (108) ein flexibles gedrucktes leitendes Material umfasst.

## Revendications

1. Dispositif (100) comprenant :
un manchon (102) conçu pour être inséré dans le pharynx d'un patient, le manchon comportant une partie d'extrémité proximale (102a), une partie d'extrémité distale (102b), une lumière (117) s'étendant de la partie d'extrémité proximale à la partie d'extrémité distale et une partie conductrice (108) espacée de la partie d'extrémité distale (102b) du manchon (102) ; et
un élément allongé (104) comportant une électrode (106) positionnée au niveau d'une surface externe de l'élément allongé,
**caractérisé en ce que**
l'élément allongé (104) est conçu pour être positionné à l'intérieur de la lumière (117) du manchon (102) de sorte que l'électrode (106) soit en communication électrique avec la partie conductrice (108) du manchon (102), et
ledit dispositif (100) étant conçu pour délivrer de l'énergie de l'électrode (106) au pharynx par l'intermédiaire de la partie conductrice (108) du manchon (102).

2. Dispositif (100) selon la revendication 1, ledit dispositif étant conçu pour stimuler électriquement les nerfs à proximité d'une zone oropharyngée du pharynx pour traiter la dysphagie.

3. Dispositif (100) selon l'une quelconque des revendications précédentes, lorsque l'élément allongé (104) est positionné à l'intérieur de la lumière (117) du manchon (102) et que le dispositif (100) est inséré dans le pharynx du patient, ledit manchon (102) empêchant un contact direct entre l'élément allongé (104) et le corps du patient et son contenu.

4. Dispositif (100) selon l'une quelconque des revendications précédentes, ladite partie conductrice (108) comprenant une électrode annulaire disposée sur une surface externe du manchon (102).

5. Dispositif (100) selon l'une quelconque des revendications précédentes, ladite électrode (106) étant conçue pour entrer en contact mécanique avec la partie conductrice (108) lorsque l'élément allongé (104) est positionné à l'intérieur de la lumière (117) du manchon (102).

6. Dispositif (100) selon l'une quelconque des revendications précédentes, ledit manchon (102) étant jetable et ledit élément allongé (104) étant réutilisable.

7. Dispositif (100) selon l'une quelconque des revendications précédentes, ledit manchon (102) comportant une extrémité distale fermée (109).

8. Dispositif (100) selon l'une quelconque des revendications précédentes, ledit élément allongé (104) comprenant un élément conducteur (113) comportant une partie d'extrémité distale couplée électriquement à l'électrode (106) et une partie d'extrémité proximale conçue pour être couplée à un générateur de courant.

9. Dispositif (100) selon la revendication 8, ledit élément conducteur (113) comprenant un fil isolé le long d'au moins une partie d'une longueur du fil.

10. Dispositif (100) selon la revendication 8 ou 9, ledit élément conducteur (113) étant positionné à l'intérieur d'un canal (107) s'étendant à travers l'élément allongé (104).

11. Dispositif (100) selon l'une quelconque des revendications précédentes, comprenant en outre une structure de retenue (110) conçue pour fixer de manière amovible la position du manchon (102) et de l'élément allongé (104) l'un par rapport à l'autre.

12. Dispositif (100) selon l'une quelconque des revendications précédentes, ledit dispositif étant conçu pour être inséré par voie nasale ou orale dans le pharynx du patient.

13. Dispositif (100) selon l'une quelconque des revendications précédentes, ledit élément allongé (104) comprenant une caméra au niveau de sa partie d'extrémité distale (102a).

14. Dispositif (100) selon l'une quelconque des revendications précédentes, ledit élément allongé (104) comprenant un élément articulable.

15. Dispositif (100) selon l'une quelconque des revendications précédentes, ladite partie conductrice (108) comprenant un matériau conducteur imprimé souple.
